# EUROPÄISCHE PATENTSCHRIFT

(11) **EP 2 986 531 B1**
(45) Veröffentlichungstag und Bekanntmachung des Hinweises auf die Patenterteilung: **08.06.2022**
(21) Anmeldenummer: 14705045.4
(22) Anmeldetag: 15.02.2014
(51) Int. Cl.: B65D 51/22, A61F 9/00

(54) **BEHÄLTNIS**
CONTAINER
RÉCIPIENT

(30) Priorität: 19.04.2013 DE 102013007063
(43) Veröffentlichungstag der Anmeldung: 24.02.2016
(73) Patentinhaber: Kocher-Plastik Maschinenbau GmbH, 74429 Sulzbach-Laufen (DE)
(72) Erfinder: SPALLEK, Michael, 55218 Ingelheim (DE); GESER, Johannes, 70839 Gerlingen (DE)
(74) Vertreter: Bartels und Partner, Patentanwälte
(86) Internationale Anmeldenummer: PCT/EP2014/000421
(87) Internationale Veröffentlichungsnummer: WO 2014/169979

(56) Entgegenhaltungen:
- WO-A2-98/55368
- DE-A1-102005 025 760
- US-A1- 2004 200 855
- US-A1- 2008 073 348

## Beschreibung

Die Erfindung betrifft ein Behältnis mit den Merkmalen im Oberbegriff von Anspruch 1.

Durch die DE 42 32 305 C1 ist eine mehrteilige Kappe für Behältnisse bekannt, insbesondere für im Blasformverfahren aus Kunststoff hergestellte und in der Form gefüllte sowie verschlossene Flaschen, auch in Ampullenform, mit einem Halsteil, auf das die Kappe aufsetzbar ist, die im Anschluss an den das Halsteil aufnehmenden Abschnitt einen mit letzterem einstückig ausgebildeten Abschnitt aufweist, der als Tropfer ausgebildet ist. Dabei beschreibt die genannte Patentschrift als weiter bekannte Lösungen Flaschen solcher Art, deren mit einem Außengewinde versehener Hals an seinem freien Ende durch ein einstückig mit dem Halsteil ausgebildetes Verschlussteil verschlossen ist, wobei eine auf das Halsteil aufgeschraubte Kappe innen mit einem zentral angeordneten Dorn als Öffnungseinrichtung versehen ist, mit dem das Verschlussteil durchstoßen werden kann. Wird nach dem Durchstoßen des Verschlussteils die Kappe abgenommen, dann kann durch die im Verschlussteil gebildete Öffnung die sich in der Flasche befindliche Flüssigkeit abgegeben werden.

Durch die DE 195 80 104 T1 ist eine Behältnislösung bekannt mit einem luftdicht abgeschlossenen Behälter, der mit einer Abdeckkappe versehen ist, wobei ein in der Kappe angebrachter Dorn zum Durchstoßen einer Membran am Halsteil des Behälters dient. Der hohle Dorn bildet eine Art Aufsatz aus, der einen Austragsdurchgangsweg definiert, um dergestalt eine geregelte Flüssigkeitsentnahme sicherzustellen. Hierfür durchstößt der Dorn als Öffnungseinrichtung die Membran, und nach dem Entfernen eines ersten Kappenteils wird der Dorn über das zweite Kappenteil in der Membran zurückgehalten, um dergestalt eine geregelte Spender- bzw. Ausgabeöffnung für das Behältnis zur Verfügung zu stellen. Das erste Kappenteil kann dann erneut dem Verschließen der Spender- bzw. Abgabeöffnung für den Behältniskörper des Behälters dienen.

Des Weiteren ist durch die US 5 954 233 A ein Behältnis bekannt, wobei für einen Öffnungsvorgang bei der bekannten Behältnislösung neben einer Rampenführung zwischen Rampenteilen eines ersten Kappenteiles und eines zweiten Kappenteiles, die betätigt eine Längszustellung des zweiten Kappenteiles in eine Rastposition auf dem Halsteil des Behältnisses bewirken, was mit einem entsprechenden Kraftaufwand bei der Betätigung einhergeht, noch stegartig vorspringende Eingriffsteile am Halsteil vorhanden sind, die in zugeordnete Ausnehmungen des zweiten Kappenteils eingreifen, für dessen drehfeste Festlegung am Halsteil, was einen Öffnungsvorgang des Behältnisses erlaubt und zwar in beiden möglichen Drehrichtungen des ersten Kappenteils bei dessen Aufschraub- oder Abschraubvorgang auf das bzw. von dem Behältnis. Vergleichbare Lösungen, jedoch mit nur einem Kappenteil oder einer Kappe, die an ihrem dem Behältniskörper zugewandten Ende einen Einstechdorn aufweisen, der jeweils als Öffnungseinrichtung beim Ein- oder Aufschrauben der Kappe auf eine entsprechende Gewindestrecke des Halsteiles des Behältniskörpers eine ihm zugewandte Verschlussmembran oder ein Gehäusedeckelteil durchsticht, ist durch die US-Schriften 3 454 196 A1 sowie US 4 723 687 A1 bekannt.

Allen vorstehend genannten Lösungen im Stand der Technik ist gemeinsam, dass diese über keine sogenannte Originalitätssicherung verfügen, die eine wichtige Funktion für dahingehende Behältnisse darstellt, indem sie eben eine mögliche Manipulation des Öffnungs-/Verschlusssystems, was nicht gewünscht ist, eindeutig dem Bediener gegenüber anzeigt oder diese Manipulation sogar verhindert.

Demgegenüber ist durch die DE 10 2005 025 760 A1 ein Behältnis mit einer zweiteilig ausgeführten Kappenlösung bekannt, dessen Behältniskörper mit einer solchen Originalitätssicherung versehen ist. Der bekannte Behältniskörper lässt sich wiederum in einfacher und kostengünstiger Weise mittels eines Blasform-, Füll- und Verschließverfahrens herstellen, wie es unter der Marke "bottelpack^{®}" in der Fachwelt bekannt geworden ist, und die Kappenteile lassen sich vorzugsweise im Spritzgussverfahren herstellen, ebenso wie die Originalitätssicherung. Gerade wenn solche Behältnisse für die Aufnahme eines sensiblen Füllgutes wie Lebensmittel oder insbesondere pharmazeutische oder kosmetische Substanzen vorgesehen sind, kommt Festlegeteilen, die bei der erstmaligen Benutzung des Behältnisses durch Lösen zugeordneter Sollbruchstellen von dem ersten Kappenteil getrennt werden müssen, um den Öffnungsvorgang überhaupt durchführen zu können, eine besonders wichtige Funktion als Originalitätssicherung zu, indem sie eine Manipulation des Öffnungs-/Verschlusssystems wie vorstehend dargelegt eben eindeutig anzeigen oder gar verhindern. Hierfür wird bei der vorstehend genannten, bekannten Lösung das Festlegeteil in Form eines Festlegeringes über Sollbruchstellen mit dem Rand des ihm zugehörigen ersten Kappenteils verbunden.

Das Festlegeteil ist auf dem Halsteil des Behältniskörpers in der Aufschraubrichtung drehbar, jedoch mittels eines einseitig wirkenden Gesperres gegen eine Drehung im Abschraubsinn festgelegt. Um die Originalitätssicherung zu brechen, d.h. die Verbindung zum ersten Kappenteil zu lösen, muss der Anwender das zunächst lose eingedrehte erste Kappenteil in Einschraubrichtung (nach unten) weiterdrehen, bis der Festlegering auf der Behälterschulter aufsetzt, so dass es zur axialen Verpressung zwischen Schulter, Festlegering und Kappenteil kommt und beim Weiterdrehen sich überlagernde axiale und torsionale Kräfte die Sollbruchstellen abreißen.

Wenn der Anwender andererseits für das erste Öffnen mit einer Drehung im Abschraubsinn beginnt, ergibt sich gegenüber den beim erstgenannten Vorgehen (Einschrauben) erforderlichen langen Wegstrecken (mit bis zu mehreren Umdrehungen) ein völlig andersartiges Löseverhalten. Da die Drehung des Festlegeringes im Abschraubsinn gesperrt ist, erfolgt das Lösen der Sollbruchstellen ohne ausgeprägte Deformation und auf kurzer Wegstrecke (kleine Drehwinkel). In diesem Fall muss der Anwender jedoch nach Lösen der Sollbruchstellen die Drehrichtung umkehren, um durch Aufschrauben des ersten Kappenteils und Mitnahme des zweiten Kappenteils mit der daran befindlichen Dornkanüle den Einstichvorgang und damit das Öffnen des Behältniskörpers durchzuführen.

Die US 2008/0073348 A1 offenbart ein Behältnis mit einem an einen Behältniskörper sich anschließenden Halsteil, auf das eine einteilige Kappe aufgebracht ist, wobei der Kappe ein Festlegeteil zugeordnet ist, das mit der Kappe über mindestens eine Sollbruchstelle verbunden ist, die durch eine relative Drehbewegung zwischen der Kappe und dem Behältniskörper oder Teilen desselben lösbar ist, wobei die jeweilige Sollbruchstelle durch Drehbewegungen lösbar ist, die sowohl in der einen als auch in der anderen Drehrichtung mit annähernd gleichem Drehwinkel und annähernd gleichem Drehmoment erfolgen, wobei das Festlegeteil die Form eines im Montagezustand das Halsteil umgebenden Ringes aufweist, wobei zwischen der Innenseite des Ringes und der Außenseite des Halsteils eine die relative Drehbewegung in beiden Drehrichtungen blockierende Anschlageinrichtung gebildet ist, wobei die Anschlageinrichtung mehrere von der Innenseite des Ringes radial vorstehende Flügelteile besitzt, die, in beiden Drehrichtungen hemmend, mit Blockadeelementen am Halsteil zusammenwirken, und wobei die Flügelteile in Form von an der Innenseite des Ringes axial verlaufenden Leisten vorgesehen sind.

Die WO 98/55368 A2 offenbart ein Behältnis mit einem an einen Behältniskörper sich anschließenden Halsteil, auf das eine einteilige Kappe aufgebracht, insbesondere aufgesteckt, ist, wobei der Kappe ein Festlegeteil zugeordnet ist, das mit der Kappe über mindestens eine Sollbruchstelle verbunden ist, die durch eine relative Drehbewegung zwischen der Kappe und dem Behältniskörper lösbar ist, wobei die jeweilige Sollbruchstelle durch Drehbewegungen lösbar ist, die sowohl in der einen als auch in der anderen Drehrichtung mit annähernd gleichem Drehwinkel und annähernd gleichem Drehmoment erfolgen, und wobei das Festlegeteil die Form eines im Montagezustand das Halsteil umgebenden Ringes aufweist.

Die US 2004/0200855 A1 offenbart eine Kappe für ein hermetisch verschlossenes Behältnis mit einem ein Gewinde aufweisenden Hals, der eine mit einer durchstechbaren Membran verschlossene Öffnung aufweist, wobei die Kappe eine Basis, einen auf ihrer Innenseite angeordneten Dorn und einen mit der Kappe einstückig ausgebildeten Sicherungsring zum Sichern der Kappe auf dem Hals aufweist, wobei die Kappe auf den Hals schraubbar ist und der Sicherungsring um den Hals drehbar ist, wenn die Kappe und der Ring zusammen in eine Richtung gedreht werden, um die Kappe von dem Ring abzutrennen, bevor der Dorn die Membran durchsticht und der Sicherungsring mit dem Hals in Eingriff gelangt, um die Kappe von dem Ring abzutrennen, wenn die Kappe in eine der einen Richtung entgegengesetzte Richtung gedreht wird.

Ausgehend von diesem Stand der Technik stellt sich die Erfindung die Aufgabe, ein Behältnis der betrachteten Art zur Verfügung zu stellen, das, bei Beibehalten der im Stand der Technik erreichten Vorteile, sich durch eine verbesserte Bedienerfreundlichkeit auszeichnet.

Erfindungsgemäß ist diese Aufgabe durch ein Behältnis gelöst, das die Merkmale des Patentanspruchs 1 in seiner Gesamtheit aufweist.

Gemäß dem kennzeichnenden Teil des Anspruchs 1 besteht eine wesentliche Besonderheit der Erfindung darin, dass die Leisten jeweils an einer oberen Übergangsstelle nur über die jeweilige Sollbruchstelle in Form einer Stegbrücke mit der Kappe an deren unterem Endbereich verbunden sind; dass die Flügelteile in Form von an der Innenseite des Ringes axial verlaufenden Leisten vorgesehen sind; und dass die Leisten mit zugeordneten, radial vorstehenden Blockadeelementen am Halsteil zusammenwirken.

Ferner ist vorgesehen, dass die jeweilige Sollbruchstelle durch sowohl in der einen als auch in der anderen Drehrichtung und mit annähernd gleichem Drehwinkel und Drehmoment erfolgende Drehbewegungen lösbar ist. Dadurch, dass das Abreißen der Originalitätssicherung mit geringem Drehwinkel, also auf kurzem Weg erfolgt, auch wenn der Anwender den Öffnungszyklus mit einer Drehbewegung im Aufschraubsinn beginnt, entsteht keine Überlagerung des für das Lösen der Sollbruchstellen notwendigen Drehmoments durch andere Drehmomente, wie durch das Einstechen der Dornkanüle, wie dies bei den bekannten Lösungen teilweise der Fall ist, wo bei dem Aufschraubvorgang das zweite Kappenteil zum Aufsetzen oder Aufschnappen auf das Halsteil und das Einstechen der Dornkanäle mitgenommen wird, bevor durch Anlaufen des Festlegeringes an der Behälterschulter der Lösevorgang erfolgt. Bei der Erfindung erfolgen demgegenüber die Vorgänge des Einstechens der Dornkanüle oder das Aufschnappen der Kappe oder des jeweiligen Kappenteils unabhängig vom Brechen der Originalitätssicherung, d.h. das Lösen der Sollbruchstellen erfolgt bei der Erfindung bei beiden Drehrichtungen mit annähernd dem gleichen Drehmoment und Drehwinkel.

Bei einer bevorzugten Ausführungsform des erfindungsgemäßen Behältnisses ist vorgesehen, dass die jeweilige Sollbruchstelle über eine Stegbrücke mit vorzugsweise geringem Querschnitt realisiert ist, die sich zwischen der Kappe und dem Festlegeteil erstreckt. Durch die Reduzierung der jeweiligen Sollbruchstelle auf eine punktförmige Trennstelle lässt sich die jeweilige Sollbruchstelle mit vergleichsweise geringen Betätigungskräften lösen und dennoch im Sinne einer wirksamen Originalitätssicherung die Kappe oder das jeweilige Kappenteil für einen Öffnungsvorgang nicht ungewollt freigeben. Demgemäß ist einem ungewollten oder unbeabsichtigten Betätigen eine wirksame Sperre vorgeschoben.

Bei einer weiteren bevorzugten Ausführungsform des erfindungsgemäßen Behältnisses ist vorgesehen, dass die Öffnungseinrichtung den Behältniskörper durch Einstechen, Einschneiden oder durch Abreißen oder Abschneiden von zumindest Teilen des Behältniskörpers öffnet oder dass eine bereits vorhandene Öffnung im Behältniskörper in ablösbarer Weise von einem Verschlusskörper verschlossen ist wie einer Siegelfolie, die auch penetrierbar ausbildbar ist, beispielsweise mittels einer als Einstechdorn ausgebildeten Öffnungseinrichtung durchstoßen wird.

Ist das Behältnis mit einer einteiligen Kappe oder eben nur einem einzelnen Kappenteil ausgestattet, kann jeweils auf der Innenseite der Kappe oder des Kappenteils ein Einstechdorn vorhanden sein, der den Behältniskörper während eines Einschraubvorganges für einen Entnahmevorgang des Behältnisinhalts aufsticht. Alternative Ausgestaltungen bestehen darin, dass die Kappe oder das jeweilige Kappenteil einen Schneid- oder Abrissring aufweist, der als Öffnungseinrichtung den Behältniskörper öffnet.

Bei einer besonders bevorzugten Ausführungsform des erfindungsgemäßen Behältnisses ist vorgesehen, dass die Kappe zumindest ein erstes Kappenteil und ein zweites Kappenteil aufweist, das sich zumindest teilweise zwischen dem ersten Kappenteil und dem Halsteil erstreckt und das mit der mindestens eine Öffnung aufweisenden Öffnungseinrichtung zum Öffnen des Behältniskörpers versehen ist, dass die Öffnung mittels des ersten Kappenteiles verschließbar ist, und dass mittels des Aufbring- oder Aufschraubvorgangs des ersten Kappenteils, dieses das zweite Kappenteil derart mitnimmt, dass die Öffnungseinrichtung das Öffnen des Behältniskörpers veranlasst.

In Abhängigkeit des jeweiligen Anwendungsfalles und abhängig von Kundenwünschen, lassen sich dergestalt zwei verschiedene Öffnungsvarianten mit nur einem Behältnis realisieren. So besteht zum einen die Möglichkeit, das bevorzugt ausgebildete Behältnis mit einer sogenannten offenen Kappenanordnung zu liefern, d. h. das erste Kappenteil ist erst teilweise, beispielsweise einen halben Gewindegang, auf dem zugeordneten Gewinde des Behältniskörpers aufgeschraubt, so dass der Anwender zunächst das erste Kappenteil weiter nach unten längs des Gewindes zu drehen hat, um dann den Behältniskörper mittels der Öffnungseinrichtung regelmäßig in Form eines Dornes zu öffnen. Hierbei verbleibt das zweite Kappenteil am Halsteil und die obere Kappe in Form des ersten Kappenteiles ist in die entgegengesetzte Richtung zur bisherigen Schraubrichtung zu drehen, um die Öffnung für eine Entnahme des bevorrateten Mediums aus dem Behälter freizugeben. Zum anderen kann das Behältnis bereits geöffnet ausgeliefert werden, d. h. das erste Kappenteil ist komplett aufgeschraubt; das zweite Kappenteil ist am Halsteil definiert festgelegt und die Öffnungseinrichtung, beispielsweise in Form des Aufstechdornes hat bereits zur Freigabe der Öffnung des Behältniskörpers diesen durchstoßen. Der Anwender des Behältnisses muss nunmehr nur noch das obere erste Kappenteil abschrauben oder abdrehen, um den Behältnisinhalt, beispielsweise in Form von bevorrateten Augentropfen, nutzen zu können. Demgemäß sind mit nur einer Anordnung kundenspezifisch zwei verschiedene Arten von Entnahmemöglichkeiten realisierbar, die jedoch in beiden Fällen durch die beschriebene Originalitätssicherung gesichert sind. Es sei an dieser Stelle betont, dass sofern von Auf- oder Abschraubvorgängen der jeweiligen Kappe oder des jeweiligen Kappenteiles die Rede ist, dieser Vorgang wiederlösbare Aufschnappvorgänge sowie Verrastungen zwischen den genannten Teilen des Behältnisses mit einschließt.

Damit der durch mehrere von der Innenseite des Ringes radial vorstehende Flügelteile, die, in beiden Drehrichtungen hemmend, mit Blockadeelementen am Halsteil zusammenwirken, ausgebildete Originalitätsring von der Kappe an den jeweiligen Sollbruchstellen in beiden Drehrichtungen gleichartig und quasi spröde abreißt, sind auch Materialien mit geringer Bruchfestigkeit und großem Elastizitätsmodul besonders geeignet, beispielsweise Styrolacrylnitril (SAN), Polystyrol (PS), Polyethylenterephthalat (PET), Polyamid (PA), Polyethylen hoher Dichte (HDPE), Polypropylen (PP) und dergleichen.

Die Leisten können einen rechteckförmigen Querschnitt aufweisen.

Alternativ kann die Anordnung auch so getroffen sein, dass Leisten mit T-förmigem, Y-förmigem oder V-förmigem Querschnitt vorgesehen sind.

Die Anschlageinrichtung kann auch in der Weise ausgebildet sein, dass an einem jeweiligen Flügelteil des Ringes eine Nut für den Eingriff eines zugeordneten Blockadeelements am Halsteil vorgesehen ist.

Die Anzahl der Blockadeelemente und der Flügelteile kann zwischen je einem und je zwölf liegen, wobei bevorzugt zwei bis vier Blockadeelemente und vier bis acht Flügelteile vorgesehen sind.

Bei besonders vorteilhaften Ausführungsbeispielen besitzt der als Originalitätssicherung dienende Ring die Form einer Krone mit einem Kranz von Durchbrüchen, die an dem dem ersten Kappenteil zugewandten Rand offen sind. Die Kronenform ermöglicht dem Anwender dank der Durchbrüche eine zusätzliche, einfache Sichtkontrolle, ob das erste Kappenteil weit genug eingeschraubt ist, um durch Mitnahme des zweiten Kappenteils den Einstichvorgang durchzuführen.

Bei besonders vorteilhaften Ausführungsbeispielen verbleibt das zweite Kappenteil am Halsteil, wobei das bei geöffnetem Behältniskörper auf dem Halsteil verbleibende zweite Kappenteil in seinem den Endbereich des Halsteils übergreifenden Glockenteil nachgiebig erweiterbar ist.

Dadurch, dass das bei geöffnetem Behältniskörper auf dem Halsteil verbleibende zweite Kappenteil in seinem den Endbereich des Halsteils übergreifenden Glockenteil nachgiebig erweiterbar ist, lässt sich der Vorgang des Aufschraubens und des Öffnens durch Mitnahme des zweiten Kappenteils mit geringem Bedarf an Drehmoment durchführen, weil dank der Nachgiebigkeit seines Glockenteils das zweite Kappenteil ohne großen Kraftbedarf sicher auf den Endbereich des Halsteils aufsetzbar ist.

Um die gewünschte Nachgiebigkeit zur Verfügung zu stellen, können im Glockenteil vom Öffnungsrand ausgehende, axiale Schlitze ausgebildet sein.

Mit Vorteil kann weiterhin vorgesehen sein, dass zur Bildung einer Schnappverbindung zwischen dem zweiten Kappenteil und dem Halsteil am Glockenteil innenseitig in Umfangsrichtung verlaufende Rippen für den Eingriff in eine Ringnut am Halsteil vorgesehen sind.

Nachstehend ist die Erfindung anhand von in der Zeichnung prinzipiell dargestellten Ausführungsbeispielen im Einzelnen erläutert. Es zeigen:
- Fig. 1: einen Längsschnitt eines ersten Ausführungsbeispiels des erfindungsgemäßen Behältnisses mit einer zweiteilig ausgebildeten Kappe, wobei der Behältniskörper schematisch vereinfacht lediglich in seinem Umriss dargestellt ist;
- Fig. 2: eine perspektivische Darstellung des ersten Ausführungsbeispiels, wobei der Zentralbereich in der Art eines Winkelschnittes dargestellt ist;
- Fig. 3: einen Teillängsschnitt lediglich des oberen Endbereichs des Halsteils des Behältniskörpers mit aufgesetztem ersten und zweiten Kappenteil der Gesamtkappe;
- Fig. 4: einen gegenüber Fig. 3 vergrößert gezeichneten Längsschnitt lediglich des zweiten Kappenteils;
- Fig. 5: in weiter vergrößerter Darstellung einen Teillängsschnitt lediglich des oberen Teils von erstem und zweitem Kappenteil;
- Fig. 6: eine Seitenansicht des gesondert dargestellten zweiten Kappenteils;
- Fig. 7: einen vergrößert und abgebrochen gezeichneten Teilquerschnitt je eines Umfangsbereichs des Halsteils mit vorspringendem Blockadeelement und des als Originalitätssicherung dienenden, ringförmigen Festlegeteils mit radial nach innen vorspringendem Flügelteil mit T-förmigem Querschnitt;
- Fig. 8 und 9: der Fig. 7 entsprechende Teilquerschnitte mit Y-förmigem bzw. V-förmigem Flügelteil;
- Fig. 10: eine den Fig. 7 bis 9 entsprechende Darstellung mit einer am jeweiligen Flügelteil des ringförmigen Festlegeteils befindlichen axialen Nut für den Eingriff eines zugeordneten Blockadeelements am Halsteil;
- Fig. 11: eine der Fig. 1 entsprechende Darstellung eines weiteren zweiten Ausführungsbeispieles mit nur einer Kappe; und
- Fig. 12: vier verschiedene Varianten von ringförmigen Festlegeteilen mit unterschiedlich ausgestalteten Fensteröffnungen.

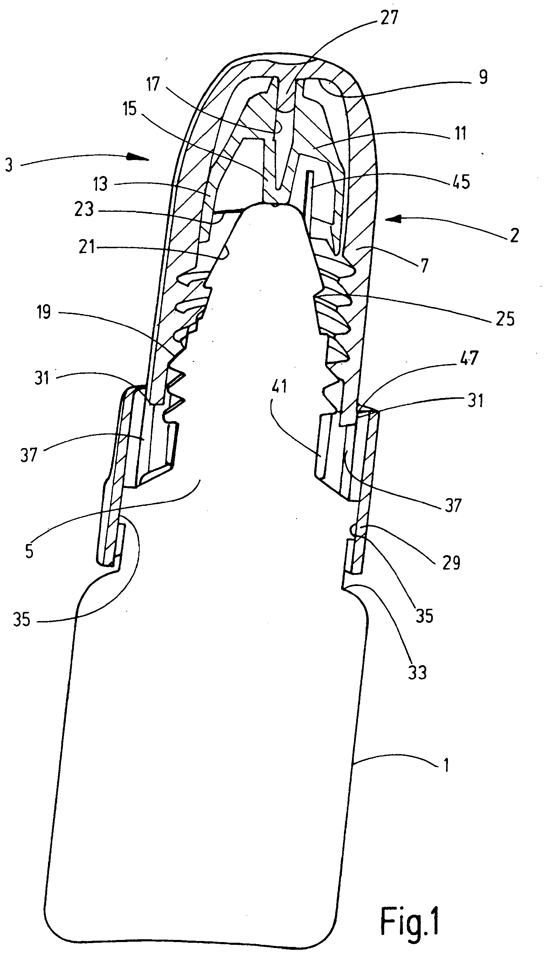

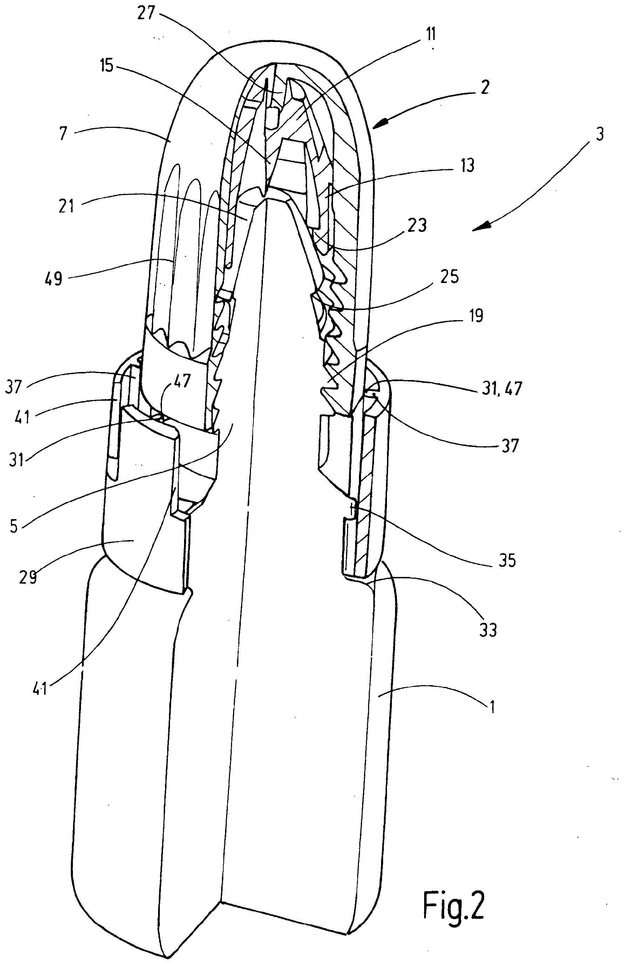

Die Fig. 1 und 2 zeigen ein erstes Ausführungsbeispiel des erfindungsgemäßen Behältnisses. Wie dort aufgezeigt, weist das Behältnis einen mit 1 bezeichneten Behältniskörper mit einer zweiteiligen Kappe 2 und mit einer als Ganzes mit 3 bezeichneten Öffnungseinrichtung auf. Der Behältniskörper 1 ist in der Art einer Ampulle aus einem Kunststoffmaterial hergestellt, in der ein nicht näher dargestelltes Fluid bevorratbar ist, beispielsweise eine medizinisch wirkende Flüssigkeit, beispielsweise in Form von Augentropfen od. dgl.. Auch andersartige Medien, etwa auch gasförmige oder pastöse Substanzen, lassen sich in den Behältniskörper 1 einbringen, beispielsweise mittels des eingangs erwähnten Blasformverfahrens, das in der Fachwelt unter der Markenbezeichnung "bottelpack^{®}" bekannt geworden ist. Die Öffnungseinrichtung 3 ist auch für andersartig hergestellte Behältniskörper 1 einsetzbar.

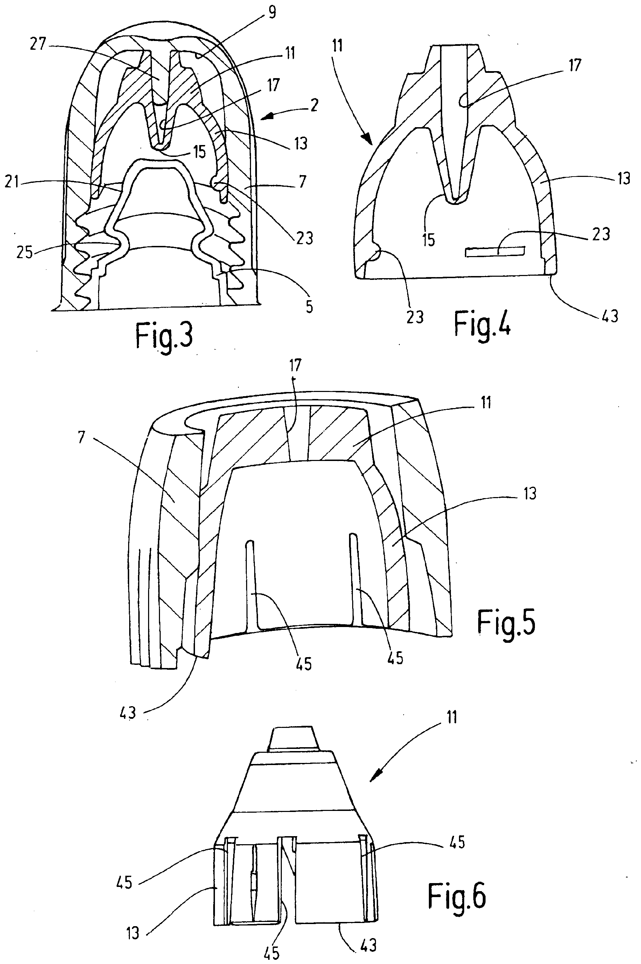

An den Behältniskörper 1 schließt sich nach oben hin ein Halsteil 5 an, auf das ein erstes Kappenteil 7 der Kappe 2 aufschraubbar ist. In das erste Kappenteil 7 ist an dessen geschlossenem oberen Ende 9 ein zweites Kappenteil 11 der Kappe 2 eingelegt, von dem in Fig. 3 bis 6 nähere Einzelheiten gezeigt sind, wobei das zweite Kappenteil 11 in Fig. 4 im Längsschnitt und in Fig. 6 in Ansicht jeweils gesondert dargestellt ist. Das zweite Kappenteil 11 ist als Öffnungselement für den Behältniskörper 1 vorgesehen und weist an seiner dem Halsteil 5 zugewandten Seite ein Glockenteil 13 auf, in dessen Zentrum eine Dornkanüle 15 gebildet ist, deren Kanüle sich über einen Kanal 17 zum oberen Ende des zweiten Kappenteils 11 fortsetzt. Bei einem Aufschrauben des ersten Kappenteils 7 auf das Gewinde 19 des Halsteils 5 wird das zweite Kappenteil 11 vom geschlossenen Ende 9 des ersten Kappenteils 7 mitgenommen, so dass das Glockenteil 13 auf das konusartige Endteil 21 des Halsteils 5 aufgeschoben wird, wobei die Dornkanüle 15 die Wand des Behältniskörpers 1 durchsticht, um eine Öffnung für eine Ausgabe des Behälterinhalts über den Kanal 17 zu bilden. Bei diesem Öffnungsvorgang wird dann auch die Dornkanüle 15 an ihrem unteren Ende für eine Fluidentnahme unter Bildung einer Kanalöffnung aufgestoßen oder diese hatte bereits von Anfang an entsprechende Fluiddurchlässe als jeweilige Abgabeöffnung in den Kanal 17.

Die Fig. 1 und 2 zeigen den Zustand vor der erstmaligen Öffnung, wobei das erste Kappenteil 7 zunächst nur lose auf das Halsteil 5 aufgesetzt ist, wobei das Kappenteil 7 beim Montagevorgang ohne Drehbewegung durch axiales Aufschieben auf das obere Ende des Gewindes 19 am Halsteil 5 aufgeschnappt ist. Wie Fig. 1 und 2 zeigen, weist das Gewinde 19 eine Sägezahnform auf, die dieses Aufschnappen ermöglicht, das Kappenteil 7 jedoch gegen eine vom Halsteil 5 weg gerichtete axiale Bewegung sichert. Wenn für den ersten Öffnungsvorgang das Kappenteil 7 aufgeschraubt wird und die Dornkanüle 15 den Einstich durchführt, wird das zweite Kappenteil 11 mit seinem Glockenteil 13 auf das konusartige Endteil 21 des Halsteils 5 aufgesetzt. Für den Verbleib auf dem Endteil 21 weist das Glockenteil 13 innenseitig mehrere vorstehende, sich in Umfangsrichtung erstreckende Rippen 23 auf, die, siehe insbesondere Fig. 3 und 4, mit einer Ringnut 25 verrasten. Bei geöffnetem Behältniskörper 1 und aufgeschraubtem ersten Kappenteil 7 ist der Kanal 17 durch einen einen Stopfen bildenden Zapfen 27 verschlossen, der am Ende 9 des Kappenteils 7 angeformt ist.

Für die Ausbildung einer Originalitätssicherung, die eine Anzeige liefert, ob sich das Behältnis noch im Originalzustand befindet oder bereits eine Manipulation der Öffnungseinrichtung 3 stattgefunden hat, ist dem ersten Kappenteil 7 ein Festlegeteil in Form eines Ringes 29 zugeordnet, der im Montage- oder Originalzustand über Sollbruchstellen 31 an dem Öffnungsrand des ersten Kappenteils 7 angeformt ist. Das Lösen dieser Verbindung durch Brechen der Sollbruchstellen 31 erfolgt durch Verdrehen des Kappenteils 7 relativ zum Ring 29. Um die Relativdrehung zu ermöglichen, ist zwischen dem Ring 29 und dem Halsteil 5 des Behältniskörpers 1 eine Anschlageinrichtung vorgesehen, die den Ring 29 gegen Verdrehen in beide Drehrichtungen sichert. Wie Fig. 1 und 2 zeigen, umgibt der Ring 29 das Halsteil 5 in dem sich an die Behälterschulter 33 anschließenden Bereich.

Bei dem in Fig. 1 und 2 gezeigten Ausführungsbeispiel weist die Anschlageinrichtung am Halsteil 5 radial vorspringende Blockadeelemente 35 auf, die in Zusammenwirkung mit von der Innenseite des Ringes 29 radial vorstehenden Flügelteilen Sperren gegen Drehbewegungen bilden. Bei dem Ausführungsbeispiel von Fig. 1 und 2 sind die Flügelteile durch Leisten 37 gebildet, die sich, mit viereckförmigem Querschnitt, in Axialrichtung entlang der Innenseite des Ringes 29 erstrecken. Während in Fig. 1 und 2 lediglich einige Blockadeelemente 35 und Leisten 37 sichtbar sind, versteht sich, dass eine Mehrzahl von Blockadeelementen 35 und Leisten 37 vorhanden ist, um eine wirksame Sperre bereits bei kleinen Drehwinkeln in beiden Drehrichtungen zu bilden. Vorzugsweise sind zwei bis vier Blockadeelemente 35 und vier bis acht Flügelteile, beispielsweise in Form der Leisten 37 vorgesehen.

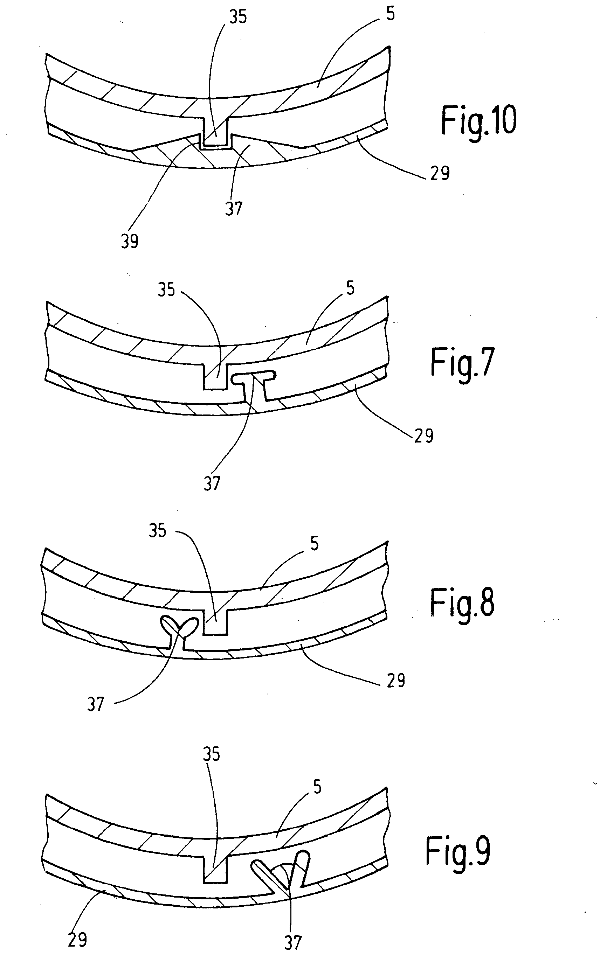

Die Fig. 7 bis 10 verdeutlichen abgewandelte Ausführungsbeispiele mit abgewandelter Bauweise der Anschlageinrichtung. Die Beispiele der Fig. 7 bis 9 unterscheiden sich gegenüber dem Ausführungsbeispiel von Fig. 1 und 2 dadurch, dass Leisten 37 mit abgewandelter Querschnittsform vorgesehen sind, nämlich T-förmige Leisten 37 in Fig. 7, Y-förmige Leisten 37 in Fig. 8 und V-förmige Leisten 37 in Fig. 9. Durch die in Umfangsrichtung weiter ausladende Querschnittsform verringert sich der jeweilige Totraum zwischen aufeinanderfolgenden Blockadeelementen 35, so dass die Blockade bereits bei kleinen Drehwinkeln in beiden Drehrichtungen wirksam ist. Das Beispiel von Fig. 10 zeigt am Ring 29 Flügelteile 37 mit einer in Axialrichtung verlaufenden Nut 39 für den Eingriff eines jeweils zugeordneten Blockadeelements 35. Bei diesem Beispiel ist der freie Drehwinkel praktisch auf Null begrenzt. Die einzelnen Blockadeelemente 35 können alternativ zumindest teilweise am Ring 29 angeordnet sein und die zusammenwirkenden Elemente oder Leisten 37 am Behältniskörper 1.

Dank der Blockade der Relativdrehung in beiden Drehrichtungen kann der Benutzer die Originalitätssicherung durch Drehen am Kappenteil 7 in beliebiger Richtung lösen, ohne mehrere Umdrehungen durchzuführen. Bei gelöster Originalitätssicherung ist der Benutzer frei, den Einstich- und Öffnungsvorgang durch weiteres Aufschrauben durchzuführen und das erste Kappenteil 7 dann zur Ausgabe von Inhaltsstoffen abzunehmen, wobei das zweite Kappenteil 11 auf das Endteil 21 des Halsteils 5 aufgeschoben und verrastet verbleibt. Nach Abgabe von Inhaltsstoffen kann durch Aufschrauben des Kappenteils 7 mittels des Zapfens 27 wieder ein Schließvorgang durchgeführt werden.

Bei dem in Fig. 1 und 2 gezeigten Ausführungsbeispiel weist der die Originalitätssicherung bildende Ring 29 die Form einer Krone mit einem Kranz von Durchbrüchen 41 auf, die an dem dem ersten Kappenteil 7 zugewandten oberen Rand offen sind. Diese Durchbrüche 41 ermöglichen dem Benutzer eine einfache Sichtkontrolle, ob die Kappenteile 7, 11 für den Einstich- und Öffnungsvorgang ausreichend weit aufgeschraubt sind.

Um das Aufsetzen des zweiten Kappenteils 11 auf das Endteil 21 des Halsteils 5 und die Sicherung durch Verrasten des Glockenteils 13 einfach und sicher zu gestalten, sind im Glockenteil 13 vom Öffnungsrand 43 ausgehend mehrere in Axialrichtung verlaufende Schlitze 45 ausgebildet. Diese verleihen dem Glockenteil 13 eine gewisse elastische Nachgiebigkeit, so dass sich das Glockenteil 13 beim Aufschieben auf das Endteil 21 radial erweitern kann, bevor die Rippen 23 durch Einschnappen in der Nut 25 die Verrastung des zweiten Kappenteils 11 auf dem Halsteil 5 bewirken.

Die Leisten 37 erstrecken sich in Blickrichtung auf die Fig. 1 und 2 gesehen für eine sichere Verankerung und Kraftübertragung in vertikaler Richtung über den gesamten Innenumfang des Ringes 29 von oben nach unten. An der oberen Übergangsstelle sind die Leisten 37 dann nur über eine geringe Stegbrücke 47 mit der äußeren Kappe 7 an deren unterem Endbereich verbunden, so dass trotz der Vielzahl an derart gebildeten Sollbruchstellen 31 über die Stegbrücken 47, die gemeinsam getrennt werden, zum Lösen der Kappe 7 vom Ring 29 nur geringe Betätigungskräfte notwendig sind. Die im Übrigen auch dünnwandig ausgebildeten Stegbrücken stellen nahezu abstandsfrei die zu lösende Verbindung zwischen dem unteren Umfassungsrand der jeweils äußeren Kappe 2 zu den leistenartigen Längsstegen 37 her. Vergleichbar der Anzahl der Leisten 37 sind auch Sollbruchstellen 31 über die genannten Stegbrücken 47 vorhanden. Sollbruchlösungen sind hier gleichfalls möglich, bei denen gegebenenfalls über eine längere Wegstrecke die jeweilige Innenseite der Leiste 37 mit der zugewandten Außenseite der Kappe 2 in lösbarer Weise verbunden ist. Insbesondere hat in Blickrichtung auf die einzelnen Figuren gesehen, die jeweilige Stegbrücke 47 eine horizontale Ausrichtung gegenüber den vertikal angeordneten Leisten 37.

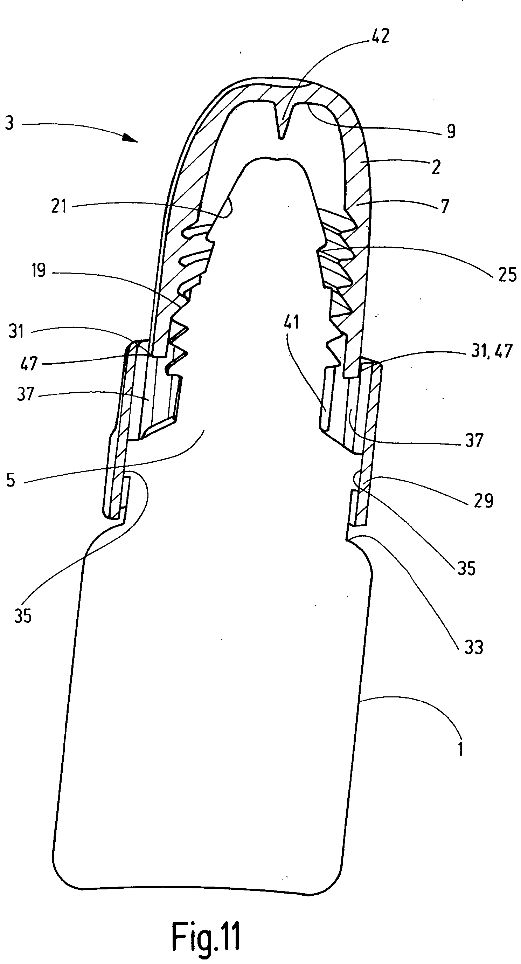

Bei der weiteren, zweiten Ausführungsform gemäß der Darstellung nach der Fig. 11, die von der zeichnerischen Darstellung vergleichbar der Lösung nach der Fig. 1 aufgebaut ist, wird anstelle einer zweiteiligen Kappe nur eine einteilige Kappe 2 eingesetzt, die über einen in diesem Fall massiv ausgebildeten Aufstechdorn 42 die Öffnung des Behältniskörpers 1 an seiner Oberseite im Bereich des Halsteiles 5 vornimmt, sobald durch einen Einschraubvorgang, in Blickrichtung auf Fig. 11 gesehen, die Kappe 2 über die zuordenbaren Gewindestrecken 19 nach unten bewegt wird. Auch im dahingehenden Fall ist die Originalitätssicherung über die Sollbruchstellen 31, 47 vorab zu lösen. Wird nach dem Aufstechen des Behältniskörpers 1 über den massiven Dorn 42 die Kappe 2 vom Behältniskörper 1 entfernt, kann über die dann nicht näher dargestellte Öffnung im Halsteil 5 ein Entnahmevorgang für den Behältnisinhalt stattfinden. Analoge Ausführungsbeispiele zu der Gestaltung nach der Fig. 11 ergeben sich durch den Einsatz weiterer einteiliger Kappen, die den Behälterkörper nunmehr nicht durch Einstechen oder Einschneiden, sondern durch Abreißen oder Abschneiden öffnen können. Auch in den dahingehenden Fällen ist dann im erfindungsgemäßen Sinne lediglich ein über die Sollbruchstellen 31 angebundener Festlegering 29 als Originalitätssicherung vorzusehen.

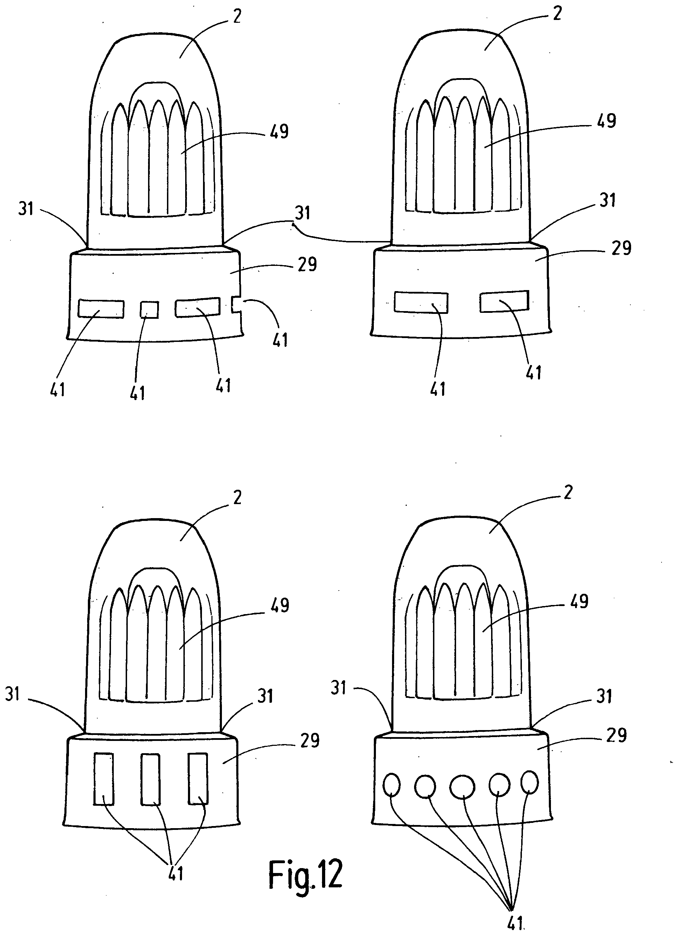

Bei den Ausführungsformen gemäß den Fig. 1 bis 11 ist der Festlegering 29 an seiner in Blickrichtung auf die Figuren gesehenen oberen Seite in der Art einer Krone mit den schlitzförmigen Durchbrüchen 41 versehen. Die Darstellung nach der Fig. 12 zeigt demgegenüber weitere Ausgestaltungen von Durchbrüchen 41 für den Ring 29. In Blickrichtung auf die Fig. 2 gesehen, ist oben links der Ring 29 mit schlitzförmigen Durchbrüchen 41 in horizontaler Ausrichtung versehen, deren Längenausdehnungen unterschiedlich gewählt sein können und eine Unterteilung in Gruppen unterschiedlicher Länge ist ermöglicht. Demgegenüber ist bei der Ausführungsform in Blickrichtung auf die Fig. 12 oben rechts die horizontal angeordneten Durchbrüche 41, die sich über den gesamten Außenumfang des Rings 29 erstrecken, gleich lang ausgebildet.

Die Ausführungsform gemäß Fig. 12 unten links zeigt eine Anordnung von Durchbrüchen 41 in Vertikalausrichtung, die zueinander einen gleichen Abstand einnehmen. Die dahingehenden vertikal verlaufenden Schlitze sind außenumfangsseitig von dem Material des Ringes 29 umgrenzt. Die verbleibende Darstellung (Fig. 12 unten rechts) zeigt nun Durchbrüche im Ring 29 in Kreisringform; hier sind auch andere Ausgestaltungen jedweder Art, auch in polygoner Form möglich. Letztendlich sollen die Durchbrüche 41 in der Art von Fensteröffnungen dem Bediener von außen her eine optische Rückmeldung geben, inwieweit die Kappe 2 in Richtung des Ringes 29 eingeschraubt ist, indem eben der untere Außenumfang der Kappe 2 bei der Einschraubbewegung nach unten in Überdeckung kommt mit der jeweiligen Durchtrittsstelle 41 und so eine optische Positionserkennung für die Kappe 2 ermöglicht. Dadurch dass die Durchbrüche 41 an mehreren Stellen entlang des Außenumfanges im Ring 29 angeordnet sind, kann man unabhängig von der Lageposition des Behältnisses 1 gegenüber dem Bediener die angestrebte optische Erkennung vornehmen. Abschließend sei noch bemerkt, dass für eine bessere Betätigung der jeweiligen Kappe 2 diese außenumfangsseitig mit einer Riffelung 49 versehen sein kann.

## Patentansprüche

1. Behältnis, insbesondere im Blasformverfahren hergestellte und in der Form gefüllte sowie verschlossene Kunststoff-Ampulle, mit einem an einen Behältniskörper (1) sich anschließenden Halsteil (5), auf das eine ein- oder mehrteilige Kappe (2) aufgebracht, insbesondere aufgesteckt, ist, wobei der Kappe (2) ein Festlegeteil (29) zugeordnet ist, das mit der Kappe (2) über mindestens eine Sollbruchstelle (31) verbunden ist, die durch eine relative Drehbewegung zwischen der Kappe (2) und dem Behältniskörper (1) oder Teilen desselben lösbar ist, wobei die jeweilige Sollbruchstelle (31) durch Drehbewegungen lösbar ist, die sowohl in der einen als auch in der anderen Drehrichtung mit annähernd gleichem Drehwinkel und annähernd gleichem Drehmoment erfolgen,
wobei das Festlegeteil die Form eines im Montagezustand das Halsteil (5) umgebenden Ringes (29) aufweist,
wobei zwischen der Innenseite des Ringes (29) und der Außenseite des Halsteils (5) eine die relative Drehbewegung in beiden Drehrichtungen blockierende Anschlageinrichtung (35, 37) gebildet ist, und
wobei die Anschlageinrichtung (35, 37) mehrere von der Innenseite des Ringes (29) radial vorstehende Flügelteile (37) besitzt, die, in beiden Drehrichtungen hemmend, mit Blockadeelementen (35) am Halsteil (5) zusammenwirken,
**dadurch gekennzeichnet,**
**dass** die Flügelteile in Form von an der Innenseite des Ringes (29) axial verlaufenden Leisten (37) vorgesehen sind; und
**dass** die Leisten (37) mit zugeordneten, radial vorstehenden Blockadeelementen (35) am Halsteil (5) zusammenwirken; und
**dass** die Leisten (37) jeweils an einer oberen Übergangsstelle nur über die jeweilige Sollbruchstelle (31) in Form einer Stegbrücke (47) mit der Kappe (2) an deren unterem Endbereich verbunden sind.

2. Behältnis nach Anspruch 1, **dadurch gekennzeichnet, dass** die jeweilige Sollbruchstelle (31) über eine Stegbrücke (47) mit vorzugsweise geringem Querschnitt realisiert ist, die sich zwischen der Kappe (2) und dem Festlegeteil (29) erstreckt.

3. Behältnis nach Anspruch 1 oder 2, **dadurch gekennzeichnet, dass** eine Öffnungseinrichtung (3) den Behältniskörper (1) durch Einstechen, Einschneiden oder durch Abreißen oder Abschneiden von zumindest Teilen des Behältniskörpers (1) öffnet oder dass eine bereits vorhandene Öffnung im Behältniskörper (1) in ablösbarer Weise von einem Verschlusskörper verschlossen ist wie einer Siegelfolie, die auch penetrierbar ausbildbar ist.

4. Behältnis nach einem der vorstehenden Ansprüche, **dadurch gekennzeichnet, dass** die Kappe (2) bei einer einteiligen Ausgestaltung einen Einstechdorn (42) oder einen Schneid- oder Abrissring aufweist, der den Behältniskörper (1) öffnet.

5. Behältnis nach einem der vorstehenden Ansprüche, **dadurch gekennzeichnet, dass** die Kappe (2) zumindest ein erstes Kappenteil (7) und ein zweites Kappenteil (11) aufweist, das sich zumindest teilweise zwischen dem ersten Kappenteil (7) und dem Halsteil (5) erstreckt und dass mit der mindestens eine Öffnung (17) aufweisenden Öffnungseinrichtung (3) zum Öffnen des Behältniskörpers (1) versehen ist, dass die Öffnung (17) mittels des ersten Kappenteils (7) verschließbar ist, und dass mittels des Aufbring- oder Aufschraubvorgangs des ersten Kappenteiles (7) dieses das zweite Kappenteil (11) derart mitnimmt, dass die Öffnungseinrichtung (3) das Öffnen des Behältniskörpers (1) veranlasst.

6. Behältnis nach einem der vorstehenden Ansprüche, **dadurch gekennzeichnet, dass** Leisten (37) mit rechteckförmigem Querschnitt vorgesehen sind.

7. Behältnis nach einem der vorstehenden Ansprüche, **dadurch gekennzeichnet, dass** Leisten (37) mit T-förmigem, Y-förmigem oder V-förmigem Querschnitt vorgesehen sind.

8. Behältnis nach einem der vorstehenden Ansprüche, **dadurch gekennzeichnet, dass** zur Bildung der Anschlageinrichtung eine in Axialrichtung im jeweiligen Flügelteil (37) des Ringes (29) verlaufende Nut (39) für den Eingriff eines zugeordneten Blockadeelements (35) am Halsteil (5) vorgesehen ist.

9. Behältnis nach einem der vorstehenden Ansprüche, **dadurch gekennzeichnet, dass** die Anzahl der Blockadeelemente (35) und der Flügelteile (37) zwischen je einem und je zwölf liegt, bevorzugt zwei bis vier Blockadeelemente (35) und vier bis acht Flügelteile (37) vorgesehen sind.

10. Behältnis nach einem der vorstehenden Ansprüche, **dadurch gekennzeichnet, dass** der Ring (29) die Form einer Krone mit einem Kranz von Durchbrüchen (41) aufweist, die an dem dem ersten Kappenteil (7) zugewandten Rand offen sind.

11. Behältnis nach einem der vorstehenden Ansprüche, **dadurch gekennzeichnet, dass** der Ring (29) ein oder mehrere fensterartige Durchbrüche (41) aufweist, die zumindest teilweise schlitzförmig, rund gestaltet oder in polygoner Form ausgebildet sind.

12. Behältnis nach Anspruch 5, **dadurch gekennzeichnet, dass** das zweite Kappenteil (11) am Halsteil (5) verbleibt, und dass das bei geöffnetem Behältniskörper (1) auf dem Halsteil (5) verbleibende zweite Kappenteil (11) in seinem den Endbereich (21) des Halsteils (5) übergreifenden Glockenteil (13) nachgiebig erweiterbar ist.

13. Behältnis nach Anspruch 12, **dadurch gekennzeichnet, dass** im Glockenteil (13) vom Öffnungsrand (43) ausgehende, axiale Schlitze (45) ausgebildet sind.

14. Behältnis nach Anspruch 12 oder 13, **dadurch gekennzeichnet, dass** zur Bildung einer Schnappverbindung des zweiten Kappenteils (11) mit dem Halsteil (5) am Glockenteil (13) innenseitig in Umfangsrichtung verlaufende Rippen (23) für den Eingriff in eine Ringnut (25) am Halsteil (5) vorgesehen sind.

## Claims

1. Container, particularly a plastic ampoule manufactured using the blow moulding process and filled and sealed in the mould, comprising a neck part (5) attached to a container body (1), a single or multi-part cap (2) being applied to, or in particular placed on, said neck part, a fixing element (29) being assigned to the cap (2), said fixing element being connected to the cap (2) by means of at least one defined breaking point (31), which can be detached by a relative rotational movement between the cap (2) and the container body (1) or parts thereof, wherein the respective defined breaking point (31) can be detached by rotational movements which take place in either direction of rotation with approximately the same angle of rotation and approximately the same torque,
wherein the fixing element has the shape of a ring (29) surrounding the neck part (5) in the assembled state, a stop device (35, 37) blocking the relative rotational movement in both directions of rotation being formed between the inside of the ring (29) and the outside of the neck part (5), and
wherein the stop device (35, 37) possesses a plurality of vane members (37) protruding radially from the inside of the ring (29), which interact with blocking elements (35) on the neck part (5), with an inhibiting effect in both directions, **characterised**
**in that** the vane members are provided in the form of axially extending strips (37) on the inside of the ring (29); and
**in that** the strips (37) interact with allocated, radially protruding blocking elements (35) on the neck part (5); and
**in that** the strips (37), are in each case connected to the cap (2), at the lower end region thereof, at an upper transition point only via the respective defined breaking point (31) in the form of a web bridge (47).

2. Container according to claim 1, **characterised in that** the respective defined breaking point (31) is created via a web bridge (47), with a preferably minimal cross-section, which extends between the cap (2) and the fixing element (29).

3. Container according to either claim 1 or claim 2, **characterised in that** an opening device (3) opens the container body (1) by piercing, cutting or by tearing or cutting off at least parts of the container body (1) or **in that** an existing opening in the container body (1) is sealed in a detachable manner by a sealing body such as a sealing film, which can also be designed to be penetrable.

4. Container according to any one of the preceding claims, **characterised in that** the cap (2), in the case of a single-part design, comprises a piercing spike (42) or a cutoff or tear-off ring that opens the container body (1).

5. Container according to any one of the preceding claims, **characterised in that** the cap (2) comprises at least one first cap part (7) and one second cap part (11), which extends at least partially between the first cap part (7) and the neck part (5) and which is furnished with the opening device (3) comprising at least one opening (17) for opening the container body (1), **in that** the opening (17) can be closed by means of the first cap part (7), and **in that** said first cap part (7) takes the second cap part (11) with it by means of the attachment or screwing operation for the first cap part (7) such that the opening device (3) causes the container body (1) to open.

6. Container according to any one of the preceding claims, **characterised in that** strips (37) with a rectangular cross-section are provided.

7. Container according to any one of the preceding claims, **characterised in that** strips (37) with a T-shaped, Y-shaped or V-shaped cross-section are provided.

8. Container according to any one of the preceding claims, **characterised in that**, in order to form the stop device, a groove (39) extending in the axial direction in the respective vane member (37) of the ring (29) is provided for engaging an assigned blocking element (35) on the neck part (5).

9. Container according to any one of the preceding claims, **characterised in that** the number of blocking elements (35) and vane members (37) is between one and twelve in each case, preferably two to four blocking elements (35) and four to eight vane members (37) are provided.

10. Container according to any one of the preceding claims, **characterised in that** the ring (29) takes the form of a crown with a circle of penetrations (41), which are open on the edge facing the first cap part (7).

11. Container according to any one of the preceding claims, **characterised in that** the ring (29) comprises one or more window-like penetrations (41), which are at least partially shaped like slits, or are round or polygonal in shape.

12. Container according to claim 5, **characterised in that** the second cap part (11) remains on the neck part (5), and **in that** the second cap part (11) remaining on the neck part (5) when the container body (1) is opened can be flexibly extended in its bell portion (13), which overhangs the end region (21) of the neck part (5).

13. Container according to claim 12, **characterised in that** axial slits (45), extending from the opening edge (43), are formed in the bell portion (13).

14. Container according to claim 12 or 13, **characterised in that**, in order to form a snapon connection between the second cap part (11) and the neck part (5), ribs (23) extending on the inside of the bell portion (13) in the circumferential direction are provided for engaging in an annular groove (25) on the neck part (5).

## Revendications

1. Récipient fabriqué notamment par le procédé de moulage par soufflage et rempli dans le moule, ainsi qu'ampoule en matière plastique fermée, comprenant une partie (5) de col, qui se raccorde au corps (1) du récipient et sur laquelle est mis, en étant notamment embroché, un capuchon (2) en une pièce ou en plusieurs pièces, dans lequel il est associé au capuchon (2) une partie (29) de fixation, qui est reliée au capuchon (2) par au moins un point (31) destiné à se rompre, qui est détachable par un mouvement relatif de rotation entre le capuchon (2) et le corps (1) du récipient ou des parties de celui-ci, le point (31) respectif destiné à se rompre étant détachable par des mouvements de rotation, qui s'effectuent tant dans un sens de rotation qu'également dans l'autre sens de rotation, avec à peu près le même angle de rotation et à peu près le même couple,
dans lequel la partie de fixation a la forme d'une bague (29) entourant la partie (5) de col à l'état de montage, dans lequel entre la face intérieure de la bague (29) et la face extérieure de la partie (5) de col est formé un dispositif (35, 37) de butée bloquant le mouvement relatif de rotation dans les deux sens de rotation, et
dans lequel le dispositif (35, 37) de butée possède plusieurs parties d'ailette (37), qui sont en saillie radialement de la face intérieure de la bague (29) et qui, en faisant obstacle dans les deux sens de rotation, coopèrent avec des éléments (35) de blocage de la partie (5) de col,
**caractérisé**
**en ce que** les parties d'ailette sont prévues sous la forme de baguettes (37) s'étendant axialement sur la face intérieure de la bague (29) ; et
**en ce que** les baguettes (37) coopèrent avec des éléments (35) associés de blocage en saillie radialement de la partie (5) de col ; et
**en ce que** les baguettes (37) sont reliées respectivement, en un point de transition supérieur, seulement par le point (31) destiné à se rompre, sous la forme d'un pont (47) d'entretoisement, au capuchon (2) à sa partie d'extrémité inférieure.

2. Récipient suivant la revendication 1, **caractérisé en ce que** le point (31) respectif destiné à se rompre est réalisé par un pont (47) d'entretoisement de section transversale, de préférence petite, qui s'étend entre le capuchon (2) et la partie (29) de fixation.

3. Récipient suivant la revendication 1 ou 2, **caractérisé en ce qu'**un dispositif (3) d'ouverture ouvre le corps (1) du récipient par décolletage, par incision ou par déchirure ou coupure d'au moins des parties du corps (1) du récipient, ou **en ce qu'**une ouverture déjà présente du corps (1) du récipient est fermée d'une manière pouvant être détachée, par un corps de fermeture comme une feuille de scellement, qui peut être aussi constituée de manière à pouvoir être transpercée.

4. Récipient suivant l'une des revendications précédentes, **caractérisé en ce que** le capuchon (2) a, dans un mode de réalisation en une seule pièce, un mandrin (42) de perçage ou une bague à couper ou à déchirer, qui ouvre le corps (1) du récipient.

5. Récipient suivant l'une des revendications précédentes, **caractérisé en ce que** le capuchon (2) a au moins une partie (7) de capuchon et une deuxième partie (11) de capuchon, qui s'étend au moins en partie entre la première partie (7) de capuchon et la partie (5) de col et qui est pourvue du dispositif (3) d'ouverture ayant au moins une ouverture (17) pour l'ouverture du corps (1) du récipient, **en ce que** l'ouverture (17) peut être fermée au moyen de la première partie (7) du capuchon et **en ce qu'**au moyen d'une opération d'application ou de vissage de la première partie (7) du capuchon, celui-ci entraîne la deuxième partie (11) du capuchon, de manière à ce que le dispositif (3) d'ouverture provoque l'ouverture du corps (1) du récipient.

6. Récipient suivant l'une des revendications précédentes, **caractérisé en ce qu'**il est prévu des baguettes (37) de section transversale rectangulaire.

7. Récipient suivant l'une des revendications précédentes, **caractérisé en ce qu'**il est prévu des baguettes (37) de section transversale en forme de T, en forme d'Y ou en forme de V.

8. Récipient suivant l'une des revendications précédentes, **caractérisé en ce que**, pour la formation du dispositif de butée, il est prévu, pour la pénétration d'un élément (35) associé de blocage de la partie (5) de col, une rainure (39) s'étendant dans la direction axiale, dans chaque partie (37) d'ailette de la bague (29).

9. Récipient suivant l'une des revendications précédentes, **caractérisé en ce que** le nombre des éléments (35) de blocage et des parties (37) d'ailette est compris entre respectivement, de préférence de deux à quatre éléments (35) de blocage et de quatre à huit parties (37) d'ailettes étant prévues.

10. Récipient suivant l'une des revendications précédentes, **caractérisé en ce que** la bague (29) a la forme d'un couronnement ayant une couronne de traversées (41), qui sont ouvertes sur le bord tourné vers la première partie (7) du capuchon.

11. Récipient suivant l'une des revendications précédentes, **caractérisé en ce que** la bague (29) a une ou plusieurs traversées (41) de type en fenêtre, qui, au moins en partie en forme de fentes, sont conformées en étant circulaires ou sont constituées en forme de polygone.

12. Récipient suivant la revendication 5, **caractérisé en ce que** la deuxième partie (11) du capuchon reste sur la partie (5) de col et **en ce que** la deuxième partie (11) du capuchon, restant sur la partie (5) de col lorsque le corps (1) du récipient est ouvert, peut être élargie en cédant élastiquement dans sa partie (13) en cloche chevauchant la région (21) d'extrémité de la partie (5) de col.

13. Récipient suivant la revendication 12, **caractérisé en ce que**, dans les parties (13) en cloche sont constituées des fentes (45) axiales partant du bord (43) d'ouverture.

14. Récipient suivant la revendication 12 ou 13, **caractérisé en ce que**, pour la formation d'une liaison à enclenchement de la deuxième partie (11) du capuchon avec la partie (5) de col, il est prévu, pour la pénétration dans une rainure (25) annulaire de la partie (5) de col, des nervures (23) s'étendant dans la direction du pourtour du côté intérieur sur la partie (13) en cloche.
